# FASCICULE DE BREVET EUROPEEN

(11) **EP 2 869 825 B1**
(45) Date de publication et mention de la délivrance du brevet: **24.08.2016**
(21) Numéro de dépôt: 13734782.9
(22) Date de dépôt: 09.07.2013
(51) Int. Cl.: A61K 33/04, A61K 45/06, A61P 15/02, A61P 31/10, A61P 13/00, A61K 35/74, C12N 1/20, A61K 35/747, A61K 9/00, A61K 47/02, A61K 9/14, A61K 9/19

(54) **UTILISATION DE THIOSULFATE POUR POTENTIALISER L'EFFET ANTI-PATHOGÈNE DES LACTOBACILLES**
VERWENDUNG VON THIOSULFAT ZUR VERSTÄRKUNG DER ANTIPATHOGENEN WIRKUNG VON LACTOBACILLUS
USE OF THIOSULPHATE IN ORDER TO POTENTIATE THE ANTI-PATHOGEN EFFECT OF LACTOBACILLUS

(30) Priorité: 09.07.2012 FR 1256570
(43) Date de publication de la demande: 13.05.2015
(73) Titulaire: Biose, 15130 Arpajon-sur-Cère (FR)
(72) Inventeur: NIVOLIEZ, Adrien, F-15130 Yolet (FR)
(74) Mandataire: Regimbeau
(86) Numéro de dépôt international: PCT/EP2013/064451
(87) Numéro de publication internationale: WO 2014/009349

(56) Documents cités:
- FR-A- 1 426 226
- US-A- 6 093 394
- US-B2- 6 919 172
- V KARTHIKEYAN ET AL: "Isolation and partial characterization of bacteriocin produced from Lactobacillus plantarum", AFRICAN JOURNAL OF MICROBIOLOGY RESEARCH, vol. 3, no. 5, 1 mai 2009 (2009-05-01), pages 233-239, XP055073122, ISSN: 1996-0808
- anonymous: "Trophigil", internet , 22 mai 2012 (2012-05-22), XP002692926, Extrait de l'Internet: URL:http://web.archive.org/web/20120807162 456/http://www.eurekasante.fr/medicaments/ vidal-famille/medicament-btropg01-TROPHIGI L.html [extrait le 2013-02-21] cité dans la demande
- MARÍA SILVINA JUÁREZ TOMÁS ET AL: "Estimation of combined effects of carbon and nitrogen sources on the growth and bacteriocin production of Lactobacillus salivarius from human source", JOURNAL OF BASIC MICROBIOLOGY, vol. 50, no. 2, 15 janvier 2010 (2010-01-15), pages NA-NA, XP055073125, ISSN: 0233-111X, DOI: 10.1002/jobm.200900122
- DE VUYST L: "Nutritional factors affecting nisin production by Lactococcus lactis subsp. actis NIZO 22186 in a synthetic medium", JOURNAL OF APPLIED BACTERIOLOGY, BLACKWELL PUBLISHING LTD., OXFORD, GB, vol. 78, no. 1, 1 janvier 1995 (1995-01-01), pages 28-33, XP008163829, ISSN: 0021-8847, DOI: 10.1111/J.1365-2672.1995.TB01669.X [extrait le 2008-03-11]

## Description

### DOMAINE DE L'INVENTION

La présente invention concerne des compositions comprenant des lactobacilles en association avec du thiosulfate, le thiosulfate potentialisant l'effet anti-pathogènes desdits lactobacilles.

Chez une femme saine, la flore urogénitale comprend près de 50 espèces différentes de microorganismes. Parmi ces microorganismes, 95% de la population est constituées de diverses souches de lactobacilles, aussi appelé « bacilles de Döderlein ». Ces lactobacilles jouent un rôle de protection contre les pathogènes par divers mécanismes : production de peroxyde d'hydrogène, production d'acide lactique, production de bactériocines, inhibition de l'adhésion et de l'expansion des pathogènes. En particulier, ces lactobacilles maintiennent un pH acide par la production d'acide lactique à partir du glycogène présent dans le mucus vaginal. Ainsi, la croissance de nombreux pathogènes de la flore vaginale tels que *Gardnerella vaginalis, Prevotella bivia, Neisseria gonorrhoeae, Mycoplasma, Mobiluncus* et surtout *Candida albicans* est inhibée.

La flore vaginale normale est ainsi principalement composée de lactobacilles formant un biofilm protecteur à la surface de la muqueuse. Les lactobacilles les plus fréquemment identifiés dans le vagin sont notamment *Lactobacillus crispatus, Lactobacillus jensenii, Lactobacillus vaginalis, Lactobacillus iners* et *Lactobacillus gasseri.*

La candidose vulvovaginale touche 70-75% des femmes en période d'activité génitale au moins une fois dans leur vie ; environ 40-50% d'entre elles présenteront un deuxième épisode. L'incidence de la candidose vulvovaginale récidivante (définie par la survenue d'au moins 4 épisodes par an, dont deux prouvés par l'examen mycologique) a été estimée à 5-8%. Cette affection bénigne a un impact très négatif sur la qualité de vie des patientes et engendre des dépenses de santé importantes. Traiter une telle pathologie est difficile du fait de la pathogénèse multifactorielle de cette affection.

Le traitement d'entretien traditionnel par antifongique oral ou vaginal doit être de 6 mois au moins, mais le taux de rechutes reste élevé avec 60-70% des femmes présentant une récidive dans les deux mois suivant l'arrêt du traitement. De plus, les effets secondaires des antifongiques sont fréquents et leur utilisation au long cours peut favoriser la survenue d'une vaginose bactérienne.

La vaginose bactérienne est due à un déséquilibre qualitatif et quantitatif de la flore vaginale normale pouvant aboutir à une disparition quasi complète des lactobacilles au profit de la flore anaérobie, et aussi à l'émergence de germes tels que *Gardnerella vaginalis* et *Atopobium vaginae.* La vaginose bactérienne est une des infections vaginales les plus fréquentes avec un taux de fréquence de 10 à 15. Cette pathologie bénigne chez la femme peut être grave pendant la grossesse, car pouvant entrainer un accouchement prématuré, des petits poids de naissance, ainsi que des avortements spontanés.

La vaginose bactérienne ainsi que les autres déséquilibres de la microflore vaginale sont communément traités par antibiothérapie. Ce traitement présente les inconvénients habituels des traitements antibiotiques et s'avère de moins en moins efficace. En outre, il vise à éliminer la flore pathogène mais détruit également la flore normale bénéfique.

### ETAT DE LA TECHNIQUE

L'administration de lactobacilles 'bénéfiques' par voie orale ou vaginale a été décrite pour promouvoir la santé vaginale. En particulier, les demandes de brevet WO 84/04675, WO 2000/035465, US 2002/0044926 et WO 2006/045475 décrivent l'administration par voie orale ou vaginale de bactéries lactiques pour promouvoir la santé vaginale et pour prévenir les récidives de la candidose vulvovaginale.

La demande de brevet WO 2010/004005 rapporte l'administration de pré-biotiques associés à un extrait végétal contenant des isoflavones, pour promouvoir le développement de la flore normale vaginale.

Les lactobacilles préférés sont les *Lactobacillus rhamnosus, Lactobacillus crispatus* et *Lactobacillus vaginalis.* Les brevets US 6,093,394, US 6,468,526 et US 7,807,440, ainsi que la demande de brevet US 2010/0151026, décrivent l'administration de souches spécifiques de *Lactobacillus crispatus.*

Ces lactobacilles peuvent être administrés sous forme lyophilisée ou en solution, et éventuellement en combinaison avec d'autres agents actifs. En effet, même si les lactobacilles ont un effet anti-pathogène réel et reconnu, celui-ci est souvent insuffisant pour combattre une infection. Des agents actifs supplémentaires, ou des agents potentialisant l'effet anti-pathogènes des lactobacilles, sont activement recherchés par la communauté scientifique.

Il a été proposé d'administrer des lactobacilles en combinaison avec un agent antimicrobien, la bactérie étant micro-encapsulée (EP 0 732 916 B1); en présence d'acide lactique et d'un extrait d'ail (EP 1 911 455 A1); en présence de gomme arabique (EP 2 211 640 A1); en présence d'estrogènes et/ou de gestagènes (WO 2010/133314) ; ou encore en présence d'un antibiotique et d'un acide gras estérifié insaturé (WO 2011/066949).

Les résultats présentés dans l'article (Nivoliez et al., 2012) indiquent que le procédé de manufacture des bactéries probiotiques pourrait jouer un rôle important dans les caractéristiques des lactobacilles préparés de façon industrielle.

Le thiosulfate de sodium est utilisé comme excipient dans de nombreuses compositions pharmaceutiques ou vétérinaires contenant des Lactobacilles, telles que les compositions TROPHIGIL, PROBIOS® et ALLBIO®. Par ailleurs, la demande de brevet CN 20091145852 décrit un médicament composé d'extraits de plantes chinoises et de thiosulfate de sodium. Ce médicament est proposé notamment pour le traitement de la gonorrhée.

Dans ces compositions, le thiosulfate de sodium est utilisé en tant qu'excipient, et non en tant qu'agent actif.

La demande de brevet américain US 2011/0008467 décrit des compositions pharmaceutiques comprenant uniquement du thiosulfate de sodium de grade pharmaceutique. Les applications thérapeutiques proposées sont les suivantes : traitement de patients ayant subi un empoisonnement au cyanure, au platine, traitement de la calcification vasculaire, et de maladies dermatologiques.

### EXPOSE DE L'INVENTION

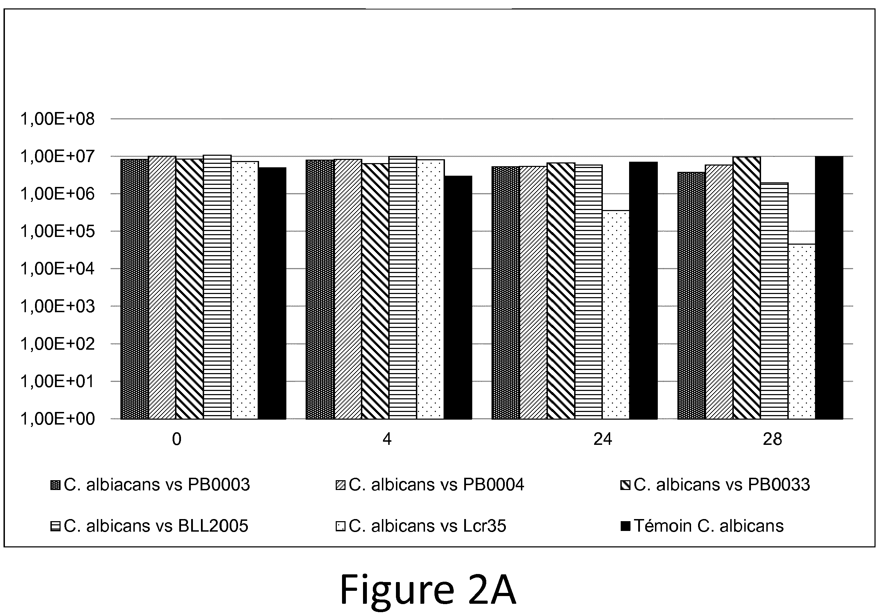

L'effet anti-pathogène des lactobacilles existe réellement mais n'est pas suffisamment fort dans la plupart des cas pour combattre l'infection (voir figure 2A), et il est donc souvent nécessaire d'utiliser d'autres principes actifs tels que des antibiotiques ou des antifongiques en complément.

La présente invention propose un moyen de potentialiser l'effet anti-pathogène des lactobacilles. Les inventeurs ont en effet découvert que l'association de thiosulfate, notamment de thiosulfate de sodium, avec des lactobacilles, permet de potentialiser l'effet anti-pathogène de ces lactobacilles, lorsque le thiosulfate est présent à une concentration d'au moins 100 mg / gramme de poudre, pour une poudre comprenant de 10⁷ à 10¹⁰ UFC/g de lactobacilles.

Cet effet 'anti-pathogène' se caractérise en particulier par une inhibition de la croissance des pathogènes vaginaux tels *que Gardnerella vaginalis, Prevotella bivia,* et surtout *Candida albicans.*

L'invention est également relative à une composition pharmaceutique comprenant au moins 100 mg/g de thiosulfate de sodium en association avec des *Lactobacillus crispatus.*

### DESCRIPTION DETAILLEE DE L'INVENTION

La présente invention est relative à l'utilisation de thiosulfate pour potentialiser l'effet anti-pathogène de bactéries lactobacille, le thiosulfate étant en quantité d'au moins 100 mg / gramme de poudre, pour une poudre comprenant de 10⁷ à 10¹⁰ UFC/g de lactobacilles.

### Définitions

Le terme « potentialiser » désigne donner une plus grande efficacité, augmenter les effets bénéfiques d'un principe actif. En particulier, l'agent potentialisateur (ici, le thiosulfate) n'a pas d'action propre contre les pathogènes, mais agit via les lactobacilles dont il potentialise l'effet anti-pathogène.

Le terme « effet anti-pathogène » désigne l'action préventive et curative des lactobacilles vis-à-vis des différents pathogènes dont la croissance peut être inhibée par la présence de lactobacilles. Cet effet s'exprime via différents mécanismes : abaissement du pH, production de peroxyde d'hydrogène (H₂O₂), production d'acide lactique et/ou d'acide acétique, production et sécrétion de bactériocines, de peptides, d'acides organiques, d'acides gras à courtes chaines, inhibition de l'adhésion et de l'expansion des pathogènes. En particulier, cet effet anti-pathogène est mesuré en taux d'inhibition de la croissance des pathogènes, ainsi qu'en taux de production de bactériocines, composés peptidiques ayant des propriétés antimicrobiennes.

Le terme « pathogènes » désigne tous les pathogènes susceptibles de s'installer dans la cavité urogénitale, et en particulier *Gardnerella vaginalis, Prevotella bivia, Neisseria gonorrhoeae,* Mycoplasma, *Neisseria gonorrhoeae, Mobiluncus, Candida albicans, Candida glabrata,* ainsi que les pathogènes susceptibles de provoquer des infections intestinales, tels que par exemple les entérobactéries (dont *Escherichia coli),* les salmonelles, les staphylocoques, *Clostridium dificile,* et les shigelles.

Le terme « lactobacilles » désigne l'ensemble des bactéries du genre *Lactobacillus,* bactéries à gram positif, immobiles, de formes et dimensions variables, et anaérobies facultatives. La plupart des lactobacilles convertissent le lactose et d'autres sucres simples en acide lactique. Les lactobacilles colonisent le vagin et le tractus gastro-intestinal, et constituent un élément important de la flore endogène intestinale et vaginale.

La présente invention est principalement relative aux lactobacilles constituant la flore vaginale ; il est cependant entendu que l'invention peut être réalisée avec d'autres types de lactobacilles tels que ceux présents dans le tractus gastro-intestinal (en particulier *Lactobacillus acidophilus, Lactobacillus reuteri, Lactobacillus salivarius, Lactobacillus gasseri, Lactobacillus casei,* et *Lactobacillus rhamnosus).*

Le terme « UFC » désigne l'unité de mesure généralement reconnue par l'homme du métier pour quantifier les bactéries capables de fonder une colonie et signifie précisément « Unité Formant Colonie ».

Le terme « thiosulfate » désigne les ions thiosulfate S₂O₃²⁻.C'est la forme basique de l'acide thiosulfurique H₂S₂O₃, instable en milieu aqueux.

Selon un aspect préféré de l'invention, les pathogènes dont la croissance est inhibée par l'association de thiosulfate et de lactobacilles appartiennent à la famille des *Candida albicans,* des *Prevotella bivia,* des *Gardnerella vaginalis,* des *Mycoplasma* ou des *Mobiluncus.*

*Candida albicans* est l'espèce de levure la plus importante et la plus connue du genre Candida. Elle provoque des infections fongiques (candidiase ou candidose) essentiellement au niveau des muqueuses digestives et gynécologiques.

*Prevotella bivia* est une bactérie anaérobie, qui provoque essentiellement des vaginoses et des infections urinaires.

*Gardnerella vaginalis* est une bactérie se présentant comme des bâtonnets pléomorphes ou des coccobacilles. C'est une bactérie retrouvée fréquemment en cas de vaginose (vaginite non spécifique) soit comme seul germe pathogène soit en association avec d'autres bactéries. Elle produit une toxine perforante qui n'affecte que les cellules humaines. On peut également la rencontrer dans le sang, les urines et le pharynx.

Le terme mycoplasmes *(Mycoplasma)* désigne une famille de plus de 100 espèces de bactéries qui sont insensibles aux familles antibiotiques telles que pénicilline ou bêta-lactame. En particulier, *Mycoplasma genitalium* est responsable d'infections génitales (urétrite, cervicite, vaginite, salpingite) et de problèmes de stérilité.

*Mobiluncus* est une bactérie gram positive, anaérobie, souvent retrouvée associée à *Gardnerella vaginalis* dans des vaginoses bactériennes.

Le terme 'vaginose' désigne un déséquilibre de la flore microbienne du vagin. Il se caractérise par la disparition des lactobacilles et la multiplication de germes anaérobies tels que *Gardnerella vaginalis.*

Le terme 'candidose' désigne une infection fongique causée par des levures du genre *Candida. Candida albicans,* l'espèce la plus fréquente, fait partie de la flore habituelle de l'oro-pharynx, du tube digestif, et peut aussi être présent en faible quantité dans la flore vaginale normale. La candidose vulvovaginale est très fréquente et peut être sujette à plusieurs récidives.

Le terme 'infection urinaire' désigne la colonisation des urines par des bactéries, ce qui se traduit le plus souvent par des signes infectieux urinaires. Les germes les plus fréquemment en cause sont *Escherichia coli* (75 % des cas), *Proteus mirabilis, Klebsiella,* tous trois des entérobactéries (bacilles gram-négatif).

Selon l'invention, ni la vaginose, ni la candidose, ni les infections urinaires ne peuvent être considérées comme des infections sexuellement transmissibles telles que la gonorrhée.

Selon un aspect de l'invention, le thiosulfate est choisi parmi le thiosulfate de sodium ou le thiosulfate de potassium. Préférentiellement, le thiosulfate de sodium est utilisé. Le thiosulfate de sodium est constitué d'ions sodium et d'ions thiosulfate. De préférence, la quantité utilisée est d'au moins 100 mg/g de poudre, préférentiellement au moins 150 mg, et plus préférentiellement au moins 200 mg de thiosulfate par gramme de poudre, cette poudre comprenant entre 10⁷ et 10¹⁰ bactéries lactobacilles (UFC). Préférentiellement, la quantité de sodium thiosulfate dans le produit final sera d'environ 230 mg/gramme de poudre comprenant 10⁷ à 10¹⁰ UFC de lactobacilles.

Selon un aspect préféré de l'invention, le thiosulfate est utilisé à une quantité d'environ 230 mg / gramme de poudre, pour une poudre comprenant entre 10⁸ et 10¹⁰ UFC de lactobacilles par gramme, et plus préférentiellement entre 10⁹ et 10¹⁰ UFC/gramme. L'homme du métier, spécialiste de la lyophilisation, saura adapter la quantité de thiosulfate ajoutée avant lyophilisation pour obtenir la quantité désirée dans la poudre obtenue après lyophilisation. En particulier, cette quantité finale de thiosulfate de sodium est obtenue par ajout de 113 g/litre de thiosulfate de sodium dans le milieu de culture bactérien, avant lyophilisation. Les exemples montrent cependant qu'un simple ajout de 1 g/litre de thiosulfate dans le milieu bactérien permet de potentialiser les effets des lactobacilles en culture.

Selon un aspect préféré de l'invention, les lactobacilles sont des *Lactobacillus rhamnosus* et/ou des *Lactobacillus crispatus.* Les souches préférées sont notamment la souche Lcr35® et la souche BLL2005.

L'invention est également relative à une composition pharmaceutique comprenant au moins 100 mg de thiosulfate, préférentiellement au moins 150 mg, et plus préférentiellement au moins 200 mg de thiosulfate par gramme de poudre, en association avec une souche de *Lactobacillus crispatus.* Selon un mode de réalisation préféré, la quantité de lactobacilles par gramme de composition sera comprise entre 10⁷ à 10¹⁰ UFC, plus préférentiellement entre 10⁸ et 10⁹ UFC/g.

De préférence, la souche de *Lactobacillus crispatus* est sous forme lyophilisée. La souche peut être le seul élément lyophilisé de la composition, mais de préférence la souche est lyophilisée dans un milieu comprenant des constituants additionnels, qui seront ajoutés avant ou après l'étape de lyophilisation. En particulier le thiosulfate de sodium peut être ajouté avant lyophilisation, notamment à une quantité de 113 g/litre de milieu bactérien. Le thiosulfate de sodium peut également être ajouté à une quantité de 1 g/litre, 5 g/litre, 10 g/litre, 20 g/litre, 30 g/litre, 50 g/litre, ou 100 g/litre de milieu bactérien.

Selon un autre aspect particulier de l'invention, la composition pharmaceutique comprend de plus une matrice de conservation et/ou des excipients bien connus de l'homme du métier, et optionnellement d'autres principes actifs ayant une action complémentaire.

En particulier, cette composition peut comprendre les principes actifs suivants : des hormones (estriol, progestérone...), des agents anti-inflammatoires et/ou des agents bactéricides et/ou des agents anti-fongiques. L'homme du métier saura déterminer quels sont les principes actifs qui peuvent être avantageusement couplés à une souche de *Lactobacillus crispatus.* Cette composition comprend également, selon un aspect spécifique de l'invention, plusieurs souches de lactobacilles.

L'invention est également relative à une composition pharmaceutique comprenant le surnageant de culture d'une souche de *Lactobacillus,* préférentiellement de *Lactobacillus crispatus,* cultivée en présence de thiosulfate, en particulier de thiosulfate de sodium, en particulier d'au moins 100 g/litre de thiosulfate de sodium dans le milieu de culture. Ce surnageant de culture contient du thiosulfate mais ne contient pas de lactobacilles. Il présente une forte activité anti-pathogène.

Comme démontré dans les exemples, la présence de thiosulfate potentialise la sécrétion de bactériocines, des composés peptidiques ayant des propriétés antimicrobiennes. Il est donc probable que le « surnageant de culture » ne contenant plus de lactobacilles, contiennent de grandes quantités de bactériocines, préalablement sécrétées par les lactobacilles.

Ces compositions pharmaceutiques sont formulées pour une administration par voie vaginale ou pour une administration par voie orale. En particulier la galénique utilisée sera sous forme de gélules, de comprimés, de crèmes, de suspensions liquides ou huileuses, ou tout autre dispositif médical approprié.

L'invention concerne également une composition pharmaceutique comprenant au moins 100 mg, préférentiellement au moins 150 mg, et plus préférentiellement au moins 200 mg de thiosulfate par gramme de poudre, en association avec une souche de *Lactobacillus crispatus,* pour son utilisation dans le traitement d'infections urogénitales telles que les vaginoses, les candidoses et les infections urinaires.

Préférentiellement, cette composition comprendra environ 230 mg de thiosulfate de sodium en association avec 10⁸ à 10⁹ UFC/g d'une souche de *Lactobacillus crispatus.*

Les modes d'administration, les posologies et les formes galéniques optimales des composés et compositions selon l'invention peuvent être déterminés selon les critères généralement pris en compte dans l'établissement d'un traitement pharmaceutique adapté à un patient, comme par exemple l'âge ou le poids corporel du patient, la gravité de son état général, la tolérance au traitement, les effets secondaires constatés. Le traitement des infections urogénitales se fera de manière préférée par administration d'une ou deux gélules de 350 mg / jour.

La présente invention est aussi relative à une composition pharmaceutique comprenant au moins 100 mg, préférentiellement au moins 150 mg, et plus préférentiellement au moins 200 mg de thiosulfate par gramme de poudre, en association avec 10⁷ à 10¹⁰ UFC d'une souche de *Lactobacillus,* pour son utilisation dans le traitement d'infections urogénitales telles que les vaginoses, les candidoses et les infections urinaires, par potentialisation de l'effet anti-pathogène des lactobacilles.

L' utilisation du thiosulfate, et en particulier du thiosulfate de sodium, pour le traitement d'infections urogénitales telles que les vaginoses, les candidoses et les infections urinaires, par potentialisation de l'effet anti-pathogène des lactobacilles est aussi décrite dans la présente demande.

Le thiosulfate pourra être utilisé en combinaison avec des lactobacilles, comme décrit ci-dessus. L'administration du thiosulfate seul dans le but de potentialiser l'effet anti-pathogène des lactobacilles présents de façon endogène dans la cavité vaginale est également décrite.

### DESCRIPTION DES FIGURES

**Figure 1****.** Influence de la concentration en sodium thiosulfate dans la formulation de la souche *L. crispatus* BLL2005 pour l'inhibition du pathogène *C*. *albicans.* Les résultats sont exprimés comme la diminution logarithmique décimale (log₁₀) du nombre d'UFC entre la numération à T0 et aux différents points de prélèvement (T=4h, 24h et 28h).
**Figure 2****.** Compositions comprenant différentes souches de lactobacilles sans (Fig 2A) ou avec (Fig 2B) du thiosulfate de sodium, pour l'inhibition du pathogène *C*. *albicans.* Les résultats sont exprimés comme la diminution logarithmique décimale (log₁₀) du nombre d'UFC entre la numération à T0 et aux différents points de prélèvement (T=4h, 24h et 28h).
**Figure 3****.** Composition comprenant la souche *L. crispatus* BLL2005 sans (Fig 3A) ou avec (Fig 3B) du thiosulfate de sodium, pour l'inhibition de différents pathogènes. Les résultats sont exprimés comme la diminution logarithmique décimale (log₁₀) du nombre d'UFC entre la numération à T0 et aux différents points de prélèvement (T4h, T24h et T28h).
**Figure 4****.** Effet du thiosulfate de sodium (TS, 1g/litre ou 3g/litre) utilisé seul sur la croissance du pathogène C. *albicans.*
**Figure 5****.** Effet de la 'formulation A', avec ou sans la présence de *L. crispatus,* sur la croissance du pathogène *C*. *albicans.* Les lots E117 et E122 contiennent des *L. crispatus ;* les lots LYO A1 et LYO A2 n'en contiennent pas.
**Figure 6****.** Co-culture de la souche Lcr35® (*L*. *rhamnosus)* formulée avec 150 mg de thiosulfate (après lyophilisation dans le produit fini), et de *Candida albicans.*

### EXEMPLES

### Exemple 1- Association de la souche L. crispatus BLL2005 et de différentes concentrations de sodium thiosulfate, pour l'inhibition du pathogène C. albicans

Des essais de co-incubation ont été réalisés entre la souche pathogène de *Candida albicans* UMIP48.72 (ATCC10231) avec la souche *L. crispatus* BLL2005, formulée avec différentes concentrations en sodium thiosulfate. La base de la formulation correspond à la formulation A (voir ci-dessous) dans laquelle nous avons fait varier la concentration en sodium thiosulfate. Cet élément est ajouté en fin de fermentation avant l'étape de lyophilisation.

La formulation A comprend les composants suivants :
- Milieu de culture: 116g/L lait, 15g/L dextrose, 10g/L d'autolysat de levure, 2ml/L tween
- Lests de Lyophilisation : 110 g/L lait, 101,5 g/L FOS, 9,5 ml/L glutamate, 5,25 g/L acide ascorbique, 113g/L sodium thiosulfate

Nous avons testé les formulations suivantes (tableau 1):

| Référence du lot | Formulation A associée avec une concentration en thiosulfate de sodium de... |
|---|---|
| E289-2005 | 0 g/l |
| E290-2005 | 10 g/l |
| E291-2005 | 25 g/l |
| E292-2005 | 50 g/l |
| E293-2005 | 75 g/l |
| E115-2005 = formulation A | 113 g/l |

### Descriptif du mode opératoire :

### Milieux de culture utilisés :

o souches de lactobacilles : bouillon/gélose de MRS (à 37°C).
o Souches pathogènes : bouillon et gélose Sabouraud (à 25°C)
o bouillon de culture de l'essai : mélange homogène des deux milieux de culture.

### Préparation des inocula :

o Travailler avec des échantillons sans blocage au froid.
o Produit probiotique : on place 0,2g dans 20 ml de bouillon MRS - à l'étuve à 37°C pendant 48h.
o Souche pathogène : on place 0,2ml dans 20 ml de bouillon Sabouraud à 25°C pendant 48h.
o La pureté des souches est contrôlée par isolement.

### Préparation des témoins :

o Pour la fabrication du témoin *pathogène,* on place 5 ml du bouillon Sabouraud inoculé (titre proche de 1.10⁸ UFC/ml) avec 5 ml de bouillon MRS non inoculé.
o Pour la fabrication du témoin *probiotique,* on place 5 ml de MRS inoculé (-titre proche de 1.10⁸ UFC/ml) avec 5 ml du bouillon Sabouraud non inoculé.

### Mise en contact des inocula :

o Placer 5 ml de la souche pathogène avec 5 ml de la souche Probiotique. On réalise les prélèvements directement dans le mélange.

### Suivi :

o Détermination de la numération du pathogène et du probiotique à T₀, T₄, T₂₄ et T₂₈ₕ
o Détermination du pH de la solution à T₀, T₄, T₂₄ et T₂₈ₕ

### Résultats :

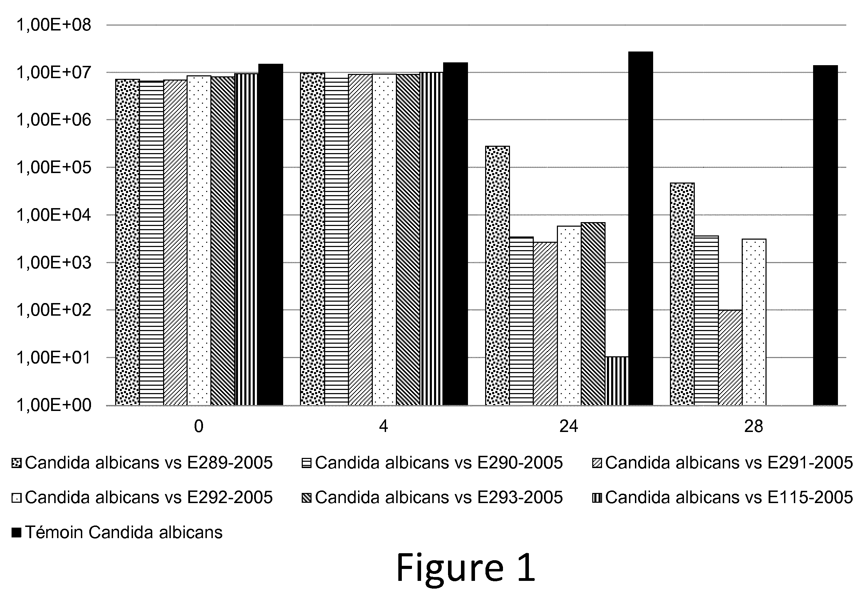

Les résultats sont présentés en figure 1 et dans le tableau 2 ci-dessous. Les résultats sont exprimés comme la diminution logarithmique décimale (log₁₀) du nombre d'UFC entre la numération à T0 et aux différents points de prélèvement (T4h, T24h et T28h).

| | **Diminution en log₁₀** | | |
|---|---|---|---|
| | **4h** | **24h** | **28h** |
| *Candida albicans vs E289*-*2005 (0 g*/*l)* | 0 | 1 | 2 |
| *Candida albicans vs E290*-*2005 (10 g*/*l)* | 0 | 3 | 3 |
| *Candida albicans vs E291*-*2005 (25 g*/*l)* | 0 | 3 | 5 |
| *Candida albicans vs E292*-*2005 (50 g*/*l)* | 0 | 3 | 3 |
| *Candida albicans vs E293*-*2005 (75 g*/*l)* | 0 | 3 | 7 |
| *Candida albicans vs E115-2005 (113 g*/*l)* | 0 | 6 | 7 |

Pour tous les essais, la numération de la souche BLL2005 restait constante au cours de la co-incubation.

### Conclusion :

Cette expérience permet de montrer que dès 10g/l de sodium thiosulfate dans la formulation A de la souche *L. crispatus* BLL2005, nous observons après 28h de co-incubation une augmentation de l'inhibition contre la souche pathogène *Candida albicans* testée d'au moins 1 log. A partir d'une concentration de 75g/l, une inhibition complète du pathogène est obtenue *in vitro.*

### Exemple 2- Association de différentes souches de Lactobacillus et de sodium thiosulfate, pour l'inhibition du pathogène C. albicans

### Souches testées :

Les souches suivants ont été testées avec ou sans la 'formulation A' comprenant 113 g/litre de sodium thiosulfate (tableau 3) :

| | | |
|---|---|---|
| PB 0003 | CIP 69.17 | *Lb jensenii T* |
| PB 0004 | CIP 101887 | *Lb reuteri T* |
| PB 0033 | CIP 105932 | *Lb vaginalis T* |
| PB 0041 | BLL2005 | *Lb crispatus* |
| PB 0039 | Lcr35® | *Lb casei variete rhamnosus* |

Le mode opératoire est équivalent à celui décrit dans l'exemple 1.

Pour tous les essais, la numération des souches de lactobacilles restaient constantes au cours de la co-incubation.

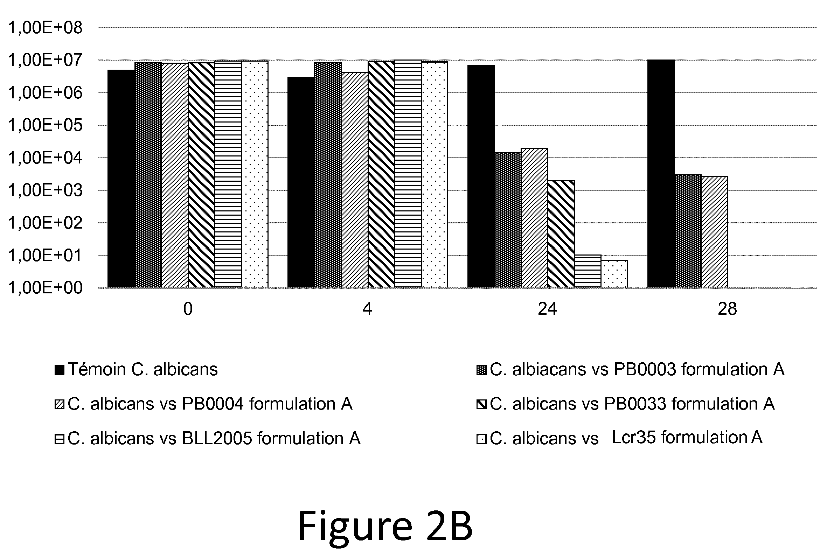

Les résultats sont montrés en figure 2. Les résultats sont exprimés comme la diminution logarithmique décimale (log₁₀) du nombre d'UFC entre la numération à T0 et aux différents points de prélèvement (T4h, T24h et T28h).

### Conclusion :

A l'état naturel, toutes les souches de *Lactobacillus* testées n'ont pas ou peu de capacités inhibitrices contre le pathogène *C*. *albicans.* (voir figure 2A).

Au contraire, lorsque la formulation A est utilisée en association avec les souches PB00033, BLL2005 et Lcr35®, une inhibition complète du pathogène après 28h de co-incubation est observée. L'association de la formulation A avec les souches PB0003 et PB0004 permet d'obtenir une inhibition du pathogène supérieure à 3 log.

Ainsi, la formulation de différentes espèces de lactobacilles avec du sodium thiosulfate permet pour les 5 souches testées d'augmenter très significativement leur capacité d'inhibition du pathogène *Candida albicans.*

### Exemple 3- Association de L. crispatus BLL2005 et de sodium thiosulfate, pour l'inhibition de plusieurs pathogènes

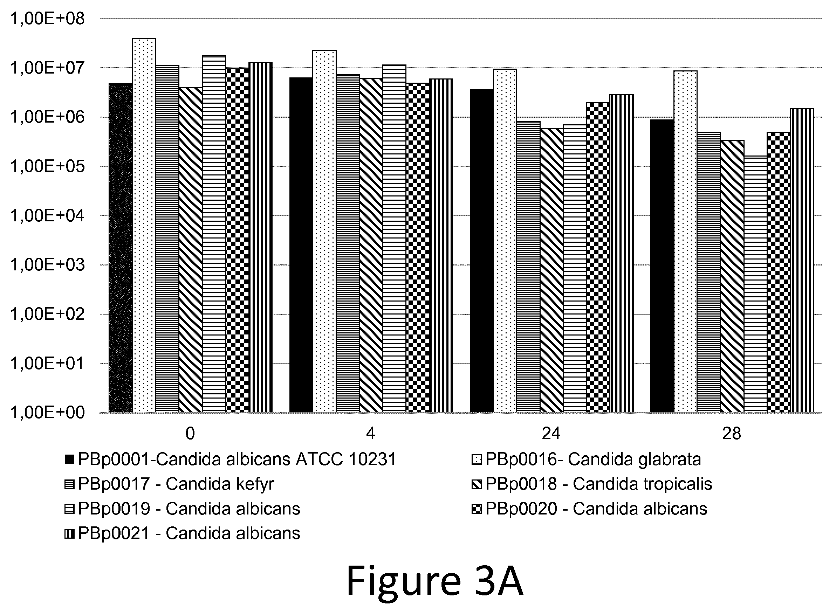

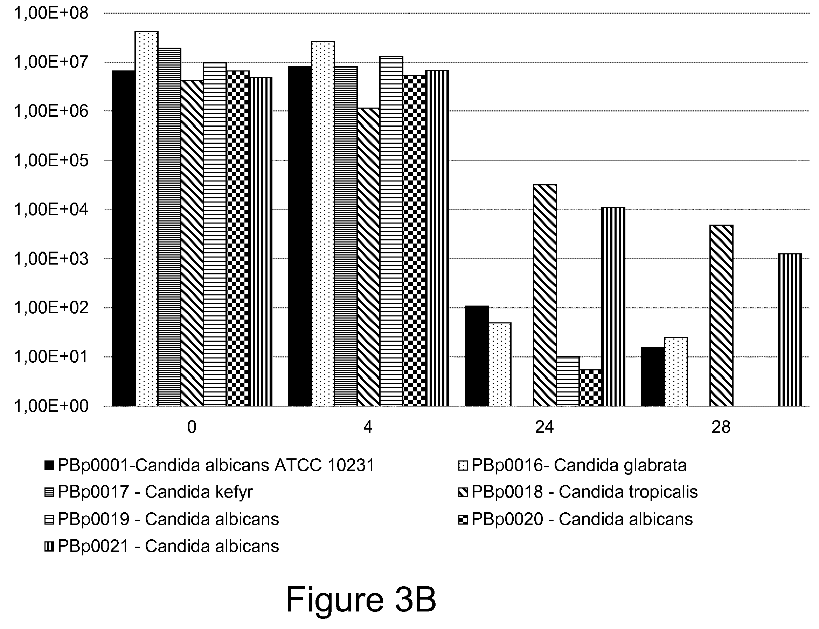

Différents essais de co-incubation ont été réalisés entre différentes souches de *Candida* avec la souche *L. crispatus* BLL2005 formulée sans thiosulfate de sodium (figure 3A) ou selon la formulation A (figure 3B).

**Tableau 4 - Souches pathogènes testées :**

| | |
|---|---|
| PBp0001 - *Candida albicans* | ATCC 10231 |
| PBp0016- *Candida glabrata* | *isolement clinique* |
| PBp0017 - *Candida kefyr* | *isolement clinique* |
| PBp0018 - *Candida tropicalis* | *isolement clinique* |
| PBp0019 - *Candida albicans* | *isolement clinique* |
| PBp0020 - *Candida albicans* | *isolement clinique* |
| PBp0021 - *Candida albicans* | *isolement clinique* |

Le mode opératoire est équivalent à celui décrit dans l'exemple 1.

Pour tous les essais, la numération des souches de lactobacilles reste constante au cours de la co-incubation. La détermination de la numération du pathogène et du probiotique se fait à T0, T4h, T24h et T28h Pour tous les essais, la numération de la souche BLL2005 reste constante au cours de la co-incubation.

Les résultats sont présentés en figure 3. Les résultats sont exprimés comme la diminution logarithmique décimale (log₁₀) du nombre d'UFC entre la numération à T0 et aux différents points de prélèvement (T4h, T24h et T28h).

### Conclusion :

La formulation A de la souche L. crispatus BLL2005 (lot testé : E115-2005) permet d'obtenir après 28h de co-incubation une inhibition supérieure ou égale à 3 log contre toutes les souches de *Candida* testées.

### Exemple 4- Préparation de la « formulation A »

La souche *BLL2005* est cultivée dans le milieu de culture suivant :
116 g/l de lait, 15 g/l de dextrose, 10 g/l d'autolysat de levure, 2 ml/l de Tween.

Après inoculation du milieu de culture, la fermentation est maintenue à une température de 37°C par un dispositif thermostaté approprié pendant 48 à 72 heures. En fin de fermentation, la culture, ayant atteint une numération supérieure ou égale à 10⁸ UFC/ml, est versée dans une cuve de mélange. La culture est agitée fortement pour briser le caillé et les adjuvants de lyophilisations suivants sont ajoutés: 110 g/l de lait, 101,5 g/l FOS, 9.5 ml/l glutamate de sodium, 5.25 g/l acide ascorbique, 113 g/l thiosulfate de sodium.

La lyophilisation est ensuite réalisée selon les conditions classiques bien connues de l'homme du métier : les bactéries sont réparties aseptiquement dans des plateaux en acier inoxydable, sont congelées rapidement à -40°C, puis soumises à une opération de sublimation à -22°C. Un étuvage à 37°C est ensuite réalisé.

Le produit est ensuite broyé et tamisé sous atmosphère contrôlée. La poudre obtenue peut être avantageusement mélangée avec 1% de stéarate de magnésium afin de faciliter le remplissage des capsules / gélules.

La poudre ainsi obtenue est répartie dans des gélules de taille n° 0 à raison de 350 mg de poudre par gélule.

### Exemple 5- Effets anti-pathogènes du surnageant de culture de la formulation A

Nous avons testé la souche de *Candida* suivante : PBp0001 - *Candida albicans* ATCC 10231.

### Milieux de culture utilisés :

o souches de Lactobacilles : bouillon/gélose de MRS (à 37°C).
o Souches pathogènes : bouillon et gélose Sabouraud (à 25°C)
o bouillon de culture de l'essai : mélange homogène des deux milieux de culture.

### Préparation des inocula :

o Travailler avec des échantillons sans blocage au froid.
o Produit probiotique :
   ▪ on place 0,2g de la formulation A dans 20 ml de bouillon MRS - à l'étuve à 37°C pendant 48h.
o Souche pathogène : on place 0,2ml dans 20 ml de bouillon Sabouraud à 25°C pendant 48h.
o La pureté des souches est contrôlée par isolement.

### Préparation des témoins :

o Pour la fabrication du témoin *pathogène,* on place 5 ml du bouillon Sabouraud inoculé (titre proche de 1.10⁸ UFC/ml) avec 5 ml de bouillon MRS non inoculé.
o Pour la fabrication du témoin *probiotique,* on place 5 ml de MRS inoculé (titre proche de 1.10⁸ UFC/ml) avec 5 ml du bouillon Sabouraud non inoculé.

### Mise en contact des inocula :

o Placer 5 ml de la souche pathogène avec 5 ml du surnageant de culture de la souche Probiotique. Le surnageant a été obtenu par une centrifugation de 10 minutes à 10 000 rpm puis une filtration à 0,22 µm. On réalise les prélèvements directement dans le mélange.

### Résultats :

Les résultats sont exprimés en diminution logarithmique décimale (log₁₀) du nombre d'UFC entre la numération à T0 et aux différents points de prélèvement (T24h, T48h et T72h).

**Tableau 5 :**

| | **Diminution en log₁₀** | | |
|---|---|---|---|
| | **24h** | **48h** | **72h** |
| *Candida albicans vs surnageant formulation A BLL2005* | 0 | 7 | 7 |
| *Témoin Candida albicans* | 0 | 0 | 0 |

### Conclusion :

Après une pré-culture de 48h sur bouillon MRS, le surnageant de la formulation A, obtenu par une centrifugation de 10 minutes à 10 000rpm puis une filtration à 0,22µm, permet d'entraîner l'inhibition complète de la souche pathogène *C*. *albicans* dès 48h de co-incubation.

### Exemple 6- Effet du thiosulfate de sodium seul sur la croissance du pathogène C. albicans

### Descriptif du protocole :

- Ensemencement de la souche 66h avant la réalisation de l'essai.
- Mise en contact de la pré culture avec le Thiosulfate de sodium.
- Détermination du titre à T0h, T24h, T48h, T72h et T96h.

### Préparation de l'essai :

- **Souche pathogène :**
   o ***Candida** albicans :* bouillon Sabouraud - **0.5 ml** du tube de travail dans 50 ml à 25°C pendant 48h.
- **Thlosulfate** : Le milieu MRS (abbréviation signifie ?)+ Thiosulfate de sodium (TS) aux différentes concentrations est préparé comme ci-dessous :
   o **Milieu MRS** + **1g/L *thiosulfate de sodium :*** On place 0,3g de TS dans 30mL de bouillon.
   o **Milieu MRS + 3g/L *thiosulfate de sodium :*** On place 0,1g de TS dans 10mL de bouillon.

### Mise en contact :

Le bouillon de culture de l'essai est un mélange homogène du milieu de culture du pathogène et du milieu MRS contenant le Thiosulfate de sodium.
- ***Candida albicans* + milieu MRS+TS (1g/L) :** on place 5mL de bouillon Sabouraud inoculé avec la souche pathogène et 5mL de milieu MRS+TS (1g/L).
- ***Candida albicans* + milieu MRS+TS (3g/L) :** on place 5mL de bouillon Sabouraud inoculé avec la souche pathogène et 5mL de milieu MRS+TS (3g/L).
- **Témoins Candida** : on place 5mL de bouillon Sabouraud inoculé avec la souche pathogène et 5mL de bouillon MRS.

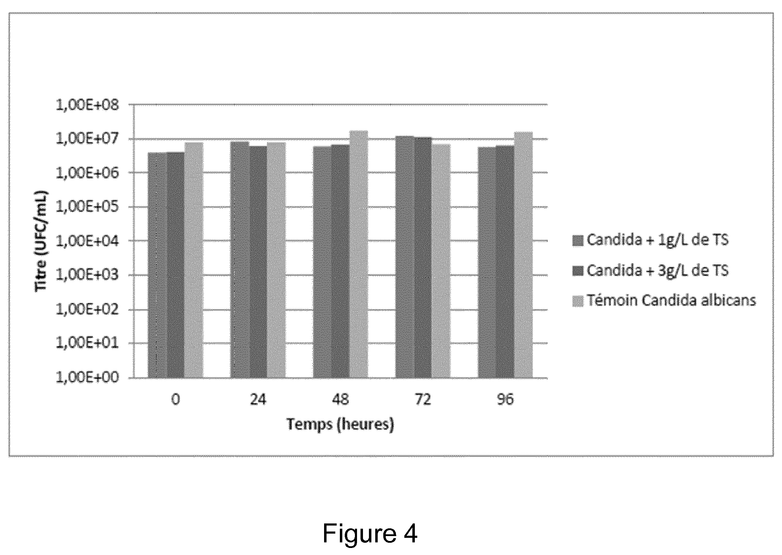

Les résultats sont présentés en figure 4.

La « formulation A » comprenant 113 g/litre de thiosulfate de sodium a également été testée pour son action anti-candida :
Préparation du témoin Candida :
   Pour la fabrication du témoin Candida, on place 5 ml de bouillon sabouraud inoculé avec la souche pathogène (titre proche de 1.108 UFC/ml) et 5 ml de bouillon MRS non inoculé.

On réalise les prélèvements et les déterminations du titre à T0, T4h, T24h et T28h.

**Tableau 6 des résultats :**

| | ***0*** | **4h** | *24h* | 28h |
|---|---|---|---|---|
| *Candida albicans* vs formulation A sans lactobacilles | 6,00E+06 | 5,50E+06 | 8,00E+06 | 9,00E+06 |
| Témoin Candida sur sabouraud | 5,00E+06 | 9,00E+06 | 1,50E+07 | 9,80E+06 |

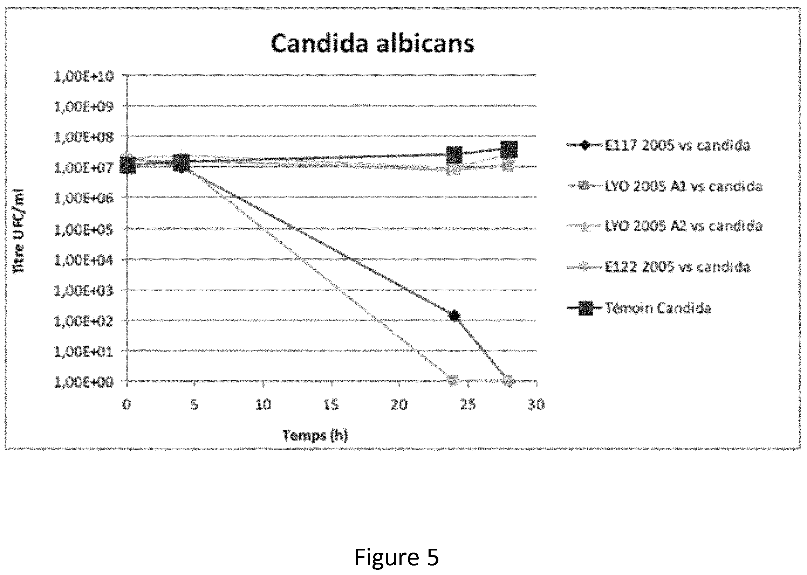

La figure 5 montre plusieurs types de culture :
- Le témoin « candida » cultivé seul
- Deux lots (E117 et E122) comprenant la souche BLL2005 dans la formulation A, telle que décrite précédemment ;
- Deux lots (LYO 2005 A1 et A2) comprenant la formulation A seule, sans bactéries.

Il est apparent que la formulation A, contenant 113 g/litre de thiosulfate de sodium, n'a aucune activité d'inhibition de la croissance des *Candida.*

Conclusion : Aucun potentiel anti-pathogène n'est observé en présence de thiosulfate de sodium, quelle que soit la concentration étudiée. Cette expérience montre que le thiosulfate ne permet pas à lui seul d'inhiber la croissance de *Candida albicans,* et qu'il n'agit qu'en tant qu'agent potentialisateur des Lactobacilles.

### Exemple 7- Co-culture de la souche Lcr35® (L. rhamnosus) formulée avec 113 g/litre de thiosulfate, et de Candida albicans, et production de bactériocines par cette souche, avec ou sans thiosulfate

### A. Inhibition de la croissance de Candida albicans en présence de la souche Lcr35® (L. rhamnosus)

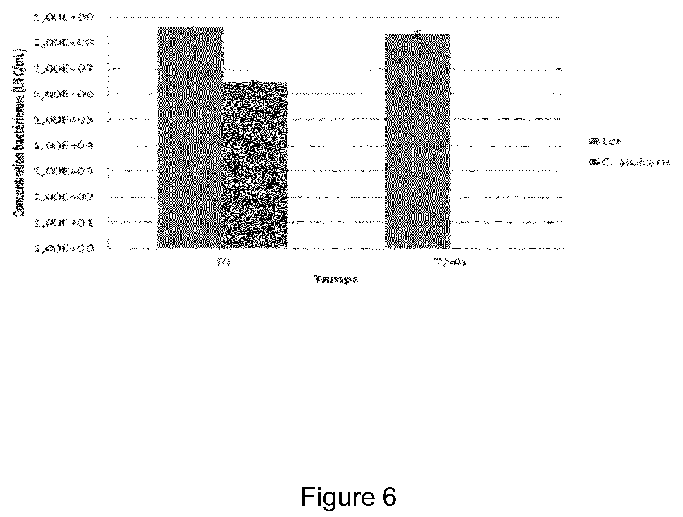

Des dénombrements ont montré une inhibition totale à T=24h de la souche pathogène avec une formulation de Lcr35® contenant 113 g/l de thiosulfate (Figure 6).

### B. Potentialisation de l'expression de gènes codant pour des bactériocines par le thiosulfate

La souche *L. rhamnosus* Lcr35® est capable d'inhiber la croissance de pathogènes. De plus, la souche formulée avec du thiosulfate est plus efficace que seule. Afin de comprendre les mécanismes qui diffèrent entre ces deux conditions, nous avons recherché des gènes impliqués dans la production de bactériocines. Chez Lcr35®, 7 gènes codant des bactériocines ont été retrouvés.

### Matériels et méthodes

Dans la littérature, différentes bactériocines sont associées à des bactéries lactiques. Les séquences des gènes codant ces dernières ont été téléchargées sur le NCBI (http://www.ncbi.nlm.nih.gov), puis alignées face aux génomes de notre souche probiotique grâce au BLAST.

Le tableau 7 ci-dessous présente les différents gènes codant des bactériocines retrouvés sur les génomes de Lcr35® :

| | |
|---|---|
| *entA* | enterocine |
| *pedC*/*papC* | pediocine |
| *spaR* | subtiline |
| *cvpA* | colicine |
| *bac1* | bactériocine inconnue |
| *spiA* | sakacine |
| *lec1* | leucocine |

### Suivi et Quantification des gènes d'intérêt

Des couples d'amorces spécifiques des gènes ciblés précédemment ont été dessinés afin de suivre leur expression par RT-PCRq. Deux cultures pures et une co-culture pour Lcr35® ont été réalisées, les premières avec la souche native probiotique, les secondes avec une formulation à 1 gLitre de thiosulfate et les dernières avec une formulation à 113 g/litre de thiosulfate, et C. *albicans.*

**Résultats : tableau 8 ci-dessous :**

| Bactériocines et gènes codants | | Lcr35® | Lcr35® + 1g/l thiosulfate | Lcr35® + 113 g/l thiosulfate |
|---|---|---|---|---|
| enterocine | *entA1* | 1 | 1 | 1 |
| | *entA2* | 1 | 3 | 6 |
| pediocine | *papC* | 1 | 3 | 5 |
| subtiline | *spaR* | 1 | 2 | 1 |
| colicine | *cvpA* | 1 | 3 | 5 |
| bactériocine inconnue | *bac1* | 1 | 2 | 2 |
| sakacine | *spiA* | 1 | 4 | 4 |
| leucocine | *lec1* | 1 | 2 | 3 |

Le suivi de l'expression de ces gènes en RT-PCR quantitative a montré une surexpression de 6 gènes codant pour des bactériocines quand la souche est formulée avec 1 g/ l de thiosulfate. Lorsque cette formulation comprend 113 g/l de thiosulfate, 3 gènes impliqués dans la sécrétion de bactériocines sont encore plus fortement surexprimés. La présence de thiosulfate pourrait donc favoriser la sécrétion de bactériocines qui participerait alors à l'inhibition de pathogènes comme C. *albicans.*

Nous avons donc montré que le thiosulfate permet de potentialiser les lactobacilles dans l'inhibition du pathogène *Candida albicans,* en permettant notamment la surproduction de bactériocines.

### REFERENCES - DOCUMENTS BREVETS

WO 84/04675
WO 2000/035465
WO 2006/045475
WO 2010/004005
WO 2010/133314
WO 2011/066949
US 2002/0044926
US 6,093,394
US 6,468,526
US 7,807,440
US 2010/0151026
US 2011/0008467
EP 0 732 916 B1
EP 1 911 455 A1
EP 2 211 640 A1
CN 20091145852

### REFERENCES BIBLIOGRAPHIQUES

Nivoliez A, Camares O, Paquet-Gachinat M, Bornes S, Forestier C, Veisseire P. "Influence of manufacturing processes on in vitro properties of the probiotic strain Lactobacillus rhamnosus Lcr35®." J Biotechnol. 2012 Aug 31;160(3-4):236-41. Epub 2012 Apr 19.

## Revendications

1. Thiosulfate pour son utilisation dans le traitement d'infections urogénitales telles que les vaginoses, les candidoses et les infections urinaires, pour potentialiser l'effet anti-pathogène de bactéries lactobacilles, le thiosulfate étant en quantité d'au moins 100 mg / gramme de poudre, pour une poudre comprenant de 10⁷ à 10¹⁰ UFC/g de lactobacilles.

2. Thiosulfate pour son utilisation selon la revendication 1, **caractérisée en ce que** le thiosulfate est choisi parmi le thiosulfate de sodium ou le thiosulfate de potassium.

3. Thiosulfate pour son utilisation selon la revendication 1 ou 2, **caractérisée en ce que** le thiosulfate est utilisé à une quantité d'environ 230 mg / gramme de poudre, pour une poudre comprenant entre 10⁷ et 10¹⁰ UFC/g.

4. Thiosulfate pour son utilisation selon la revendication 1. **caractérisée en ce que** les lactobacilles sont des *Lactobacillus rhamnosus* et/ou des *Lactobacillus crispatus.*

5. Composition pharmaceutique comprenant au moins 100 mg de thiosulfate par gramme, en association avec une souche de *Lactobacillus crispatus.*

6. Composition pharmaceutique comprenant du surnageant de culture d'une souche de *Lactobacillus* cultivée en présence de thiosulfate de sodium, ledit surnageant ne contenant pas de lactobacilles.

7. Composition pharmaceutique selon la revendication 5 ou 6, formulée pour une administration par voie vaginale ou par voie orale.

8. Composition selon l'une des revendications 6 à 7, pour son utilisation dans le traitement d'infections urogénitales telles que les vaginoses, les candidoses et les infections urinaires.

9. Composition pharmaceutique comprenant au moins 100 mg de thiosulfate par gramme, en association avec 10⁷ à 10¹⁰ UFC/g d'une souche de *Lactobacillus,* pour son utilisation dans le traitement d'infections urogénitales telles que les vaginoses, les candidoses et les infections urinaires, par potentialisation de l'effet anti-pathogène des lactobacilles.

## Patentansprüche

1. Thiosulfat für seine Verwendung bei der Behandlung von Urogenitalinfektionen wie die Vaginosen, die Candidosen und die Harnwegsinfektionen zur Potentialisierung der antipathogenen Wirkung von Lactobazillen, wobei das Thiosulfat mengenmäßig mindestens 100 mg/Gramm Pulver beträgt bei einem Pulver, das 10⁷ bis 10¹⁰ UFC/g Lactobazillen umfasst.

2. Thiosulfat für seine Verwendung nach Anspruch 1, **dadurch gekennzeichnet, dass** das Thiosulfat aus dem Natriumthiosulfat oder dem Kaliumthiosulfat ausgewählt ist.

3. Thiosulfat für seine Verwendung nach Anspruch 1 oder 2, **dadurch gekennzeichnet, dass** das Thiosulfat in einer Menge von zirka 230 mg/Gramm Pulver verwendet wird bei einem Pulver, das zwischen 10⁷ und 10¹⁰ UFC/g umfasst.

4. Thiosulfat für seine Verwendung nach Anspruch 1, **dadurch gekennzeichnet, dass** die Lactobazillen *Lactobacillus rhamnosus* und/oder *Lactobacillus crispatus* sind.

5. Pharmazeutische Zusammensetzung, umfassend mindestens 100 mg Thiosulfat pro Gramm in Kombination mit einem Stamm von *Lactobacillus crispatus.*

6. Pharmazeutische Zusammensetzung, umfassend Kulturüberstand eines Stamms von *Lactobacillus,* der bei Anwesenheit von Natriumthiosulfat kultiviert wurde, wobei der Überstand keine Lactobazillen enthält.

7. Pharmazeutische Zusammensetzung nach Anspruch 5 oder 6, formuliert für eine Verabreichung auf vaginalem Weg oder auf oralem Weg.

8. Zusammensetzung nach einem der Ansprüche 5 bis 7 für ihre Verwendung bei der Behandlung von Urogenitalinfektionen wie die Vaginosen, die Candidosen und die Harnwegsinfektionen.

9. Pharmazeutische Zusammensetzung, umfassend mindestens 100 mg Thiosulfat pro Gramm in Kombination mit 10⁷ bis 10¹⁰ UFC/g eines Stamms von *Lactobacillus* für ihre Verwendung bei der Behandlung von Urogenitalinfektionen wie die Vaginosen, die Candidosen und die Harnwegsinfektionen durch Potentialisierung der antipathogenen Wirkung der Lactobazillen.

## Claims

1. Thiosulfate for its use in the treatment of urogenital infections such as vaginosis, candidosis and urinary tract infections, to potentiate the anti-pathogenic effect of lactobacilli bacteria, the thiosulfate being in a quantity of at least 100 mg/gram of powder, for a powder including from 10⁷ to 10¹⁰ CFU/g of lactobacilli.

2. Thiosulfate for its use according to claim 1, **characterized in that** the thiosulfate is selected from sodium thiosulfate or potassium thiosulfate.

3. Thiosulfate for its use according to claim 1 or 2, **characterized in that** the thiosulfate is used in a quantity of about 230 mg/gram of powder, for a powder including between 10⁷ and 10¹⁰ CFU/g.

4. Thiosulfate for its use according to claim 1, **characterized in that** the lactobacilli are *Lactobacillus rhamnosus* and/or *Lactobacillus crispatus.*

5. Pharmaceutical composition including at least 100 mg of thiosulfate per gram, in combination with a *Lactobacillus crispatus* strain.

6. Pharmaceutical composition including the culture supernatant of a *Lactobacillus* strain cultivated in the presence of sodium thiosulfate, said supernatant not containing lactobacilli.

7. Pharmaceutical composition according to claim 5 or 6, formulated for vaginal or oral administration.

8. Composition according to one of claims 5 to 7, for its use in the treatment of urogenital infections such as vaginosis, candidosis and urinary tract infections.

9. Pharmaceutical composition including at least 100 mg of thiosulfate per gram, in combination with 10⁷ to 10¹⁰ CFU/g of a *Lactobacillus* strain, for its use in the treatment of urogenital infections such as vaginosis, candidosis and urinary tract infections, by potentiation of the anti-pathogenic effect of lactobacilli.
